(19) Europäisches Patentamt
European Patent Office
Office européen des brevets

(11) Numéro de publication : **0 391 754 B1**

(12) # FASCICULE DE BREVET EUROPEEN

(45) Date de publication du fascicule du brevet : **15.12.93 Bulletin 93/50**

(51) Int. Cl.$^5$ : **A61K 9/127**

(21) Numéro de dépôt : **90400419.9**

(22) Date de dépôt : **15.02.90**

(54) **Composition pour favoriser la cicatrisation.**

(30) Priorité : **06.04.89 FR 8904526**

(43) Date de publication de la demande : **10.10.90 Bulletin 90/41**

(45) Mention de la délivrance du brevet : **15.12.93 Bulletin 93/50**

(84) Etats contractants désignés : **AT BE CH DE DK ES GB GR IT LI NL SE**

(56) Documents cités :
**WO-A-87/06460**
**FR-A- 2 597 367**

(73) Titulaire : **L'OREAL**
**14, Rue Royale**
**F-75008 Paris (FR)**

(72) Inventeur : **Zysman, Alexandre**
**6, rue George Eastmann**
**F-75013 Paris (FR)**
Inventeur : **Sebag, Henri**
**26, rue Erlanger**
**F-75016 Paris (FR)**
Inventeur : **Vanlerberghe, Guy**
**40, rue du Général de Gaulle, Montjay-la-Tour**
**F-77410 Villevaude (FR)**
Inventeur : **Handjani, Rose-Marie**
**17bis, rue Campagne-Première**
**F-75014 Paris (FR)**
Inventeur : **Ribier, Alain**
**2, boulevard Jourdan**
**F-75014 Paris (FR)**
Inventeur : **Noblet, Jean-Paul**
**8, rue Marc Seguin**
**F-75018 Paris (FR)**

(74) Mandataire : **Peuscet, Jacques et al**
**Cabinet Peuscet 68, rue d'Hauteville**
**F-75010 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue Saint-Denis, 75001 PARIS

## Description

La présente invention concerne une composition pour favoriser la cicatrisation des tissus animaux vivants, en particulier la cicatrisation dermique chez l'homme.

On sait qu'il est très important d'obtenir une cicatrisation rapide des plaies ayant atteint le derme, et d'obtenir rapidement des cicatrices ayant une force de rupture élevée afin d'éviter toute réouverture de la plaie. La mobilité des patients après des opérations ou des accidents est ainsi plus rapidement assurée.

Dans le brevet FR-A-2 597 367, on a déjà décrit des compositions pour application topique sur la peau, ces compositions comportant dans une phase aqueuse de dispersion des vésicules fermées délimitées par une ou plusieurs bi-couches lipidiques ; parmi les lipides amphiphiles utilisables pour constituer ces vésicules, on a cité certains éthers de polyglycérol qui peuvent être associés à divers additifs, notamment du cholestérol. Ces vésicules, dont la paroi est une structure lamellaire, renferment intérieurement une phase aqueuse encapsulée, qui peut contenir divers agents actifs hydrosolubles, notamment des agents cicatrisants. Dans le brevet WO-A- 87 06460, on a également décrit des compositions pour application topique comportant des vésicules du même type que ci-dessus défini et l'on a indiqué que certains agents actifs liposolubles pouvaient aussi être incorporés dans les parois des vésicules. Mais l'effet topique de ces compositions de l'état de la technique est essentiellement fonction des agents actifs introduits dans les vésicules, aucun effet propre n'étant indiqué pour les vésicules en cause.

Selon la présente invention, il a été trouvé que certaines vésicules non-ioniques permettaient d'accélérer notablement la cicatrisation.

La présente invention concerne une composition pour application topique sur la peau comportant, dans une phase aqueuse D, des vésicules encapsulant une phase aqueuse E, lesdites vésicules étant obtenues à partir d'au moins un lipide non-ionique dérivé de polyglycérol associé à du cholestérol, la concentration des lipides vésiculaires dans la composition étant comprise entre 5 et 25 % en poids, caractérisée par le fait que, pour conférer à ladite composition une action cicatrisante en l'absence de tout agent de cicatrisation dans les phases aqueuses E ou D, le(s) dérivé(s) de polyglycérol est(sont) choisi(s) dans le groupe formé par ceux de formule :

$$RO\{CH_2\text{-}CHOH\text{-}CH_2O\}_{\overline{2}}H \qquad (I)$$

formule dans laquelle R représente soit un radical alkyle en $C_{16}$-$C_{18}$ ou un mélange de plusieurs de ces radicaux, soit un radical R'CO, R' étant un radical alkyle en $C_{15}$ -$C_{17}$ ou un mélange de plusieurs de ces radicaux, ledit lipide non-ionique étant associé à du cholestérol et la concentration des lipides vésiculaires totaux dans la composition étant de 5 à 25 % en poids, de préférence, de 6 à 1Ø % en poids.

Le lipide non-ionique de formule (I) peut avantageusement être également associé à du dicétylphosphate ou du tétradécylphosphate, notamment sous forme de sel de sodium, en complément au cholestérol.

Il convient de préciser que les lipides vésiculaires totaux sont constitués par le(s) lipide(s) non-ionique(s) de formule (I), le cholestérol et, éventuellement, le dicétylphosphate ou le tétradécylphosphate.

Les vésicules sont, de préférence, obtenues à partir d'un mélange de lipides contenant en poids 4Ø à 75 % de lipides de formule (I), 2Ø à 5Ø % de cholestérol et Ø à 1Ø %, de préférence 5 à 1Ø %, d'un dicétylphosphate ou d'un tétradécylphosphate.

Les vésicules ont, de préférence, des diamètres moyens compris entre 25 et 5.ØØØ nm, notamment entre 1ØØ et 5ØØ nm et ils présentent avantageusement un coefficient de polydispersité inférieur à Ø,4.

Les vésicules non-ioniques sont, de façon connue, délimitées par une ou plusieurs couches bimoléculaires ou multimoléculaires d'un lipide non-ionique, qui encapsulent une phase aqueuse E. Elles sont dispersées dans une phase aqueuse de dispersion D. Selon la présente invention, les phases aqueuses E et D peuvent être différentes mais sont, de préférence, identiques.

Selon l'invention, la phase aqueuse E et/ou la phase aqueuse D peuvent contenir au moins un additif cosmétique tel que notamment des hydratants, des adoucissants, des agents gélifiants, des conservateurs, des filtres et/ou au moins un actif pharmaceutique tel que des antibiotiques ou des agents anti-inflammatoires.

Les feuillets lipidiques peuvent contenir au moins un additif liposoluble cosmétique et/ou au moins un additif liposoluble pharmaceutlquement actif.

Les vésicules non-ioniques des compositions selon l'invention sont préparées par tout procédé connu permettant d'obtenir une dispersion de vésicules en phase aqueuse, dispersion que l'on soumet ensuite éventuellement à un traitement aux ultra-sons pour réduire la taille des vésicules. Un des procédés de production de vésicules proposé par Bangham (J.Mol. Biol.,13, 238 (1965)) consiste à former sur une surface, par évaporation du solvant d'une solution de lipides, un film mince de substance lipidique sur ladite surface, à mettre la surface revêtue du film en contact avec la phase aqueuse et à agiter pour obtenir une dispersion. Selon FR-A-2 221 122, on a aussi proposé d'ajouter un lipide une phase aqueuse, de chauffer légèrement le mélange puis de l'agiter énergiquement par secousses. Selon FR-A-2 315 991, on met en contact un lipide susceptible de gonfler dans l'eau et une phase aqueuse pour former une phase lamellaire, on ajoute ensuite une phase de dispersion et on agite énergiquement. D'autres procédés sont décrits par ailleurs, notamment dans FR-A-2 543 Ø18 et peuvent aussi être utilisés. Pour modifier la phase aqueuse E, on Introduira les additifs convenables au cours de la phase de gonflement.

Les compositions selon l'invention sont administrées par voie topique sur la partie de la peau comportant la plaie. Elles sont, par exemple, appliquées à raison de 2 à 8 mg/jour/cm2.

La présente invention a également pour objet l'utilisation, pour favoriser la cicatrisation, d'une composition telle que ci-dessus définie.

Les essais ci-après, donnés à titre purement illustratif et non limitatif, permettront de mieux comprendre l'invention.

Les différents essais ont été effectués sur des lots de 4Ø rats wistar femelles de 23Ø g, sur le dos desquels on a effectué, au jour J, une incision dermique longue de 1 cm. On a ensuite effectué cinq applications par voie topique du produit de cicatrisation testé, au cours de chacun des jours J+5 à J+9. Les applications ont été de Ø,2 ml/jour/rat pour une surface de 5Ø cm2.

La mesure des forces de rupture des cicatrices a ensuite été effectuée au jour J+12 à l'aide du dynamomètre Instron. On a calculé, à partir de la force de rupture, l'activité du produit traité qui est exprimée en % d'augmentation de la force de rupture par rapport à un essai témoin sans application de la composition. L'activité du témoin est donc Ø % par définition.

EXEMPLE 1

Les essais ont été effectués avec des compositions contenant 8 % de lipides vésiculaires totaux dans l'eau. Les vésicules ont été préparées par le procédé décrit dans le brevet français 2 315 991 à partir de mélanges lipidiques contenant en poids 47,5 % de lipides non-ioniques soit A, soit B, soit C, 47,5 % de cholestérol et 5 % de dicétylphosphate de sodium.

Le lipide non-ionique :

A a pour formule $R-O(CH_2-CHOH-CH_2O)_2H$
où R est $C_{16}H_{33}$
B a pour formule

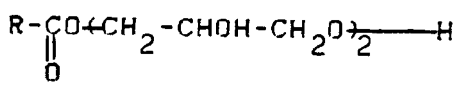

où R est $C_{15}H_{31}$
C a pour formule

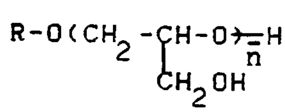

où R est $C_{16}H_{33}$ et
où $\bar{n}$ (valeur moyenne statistique) = 3

Il faut noter que les compositions contenant des vésicules formées avec les lipides non-ioniques A et B sont incluses dans la présente invention et que celles contenant des vésicules formées avec le lipide non-ionique C ne fait pas partie de l'invention.

On a obtenu les résultats donnés dans le tableau I (page 6). Les vésicules préparées avec le lipide C donnent un résultat non significatif compte-tenu des imprécisions de mesure. Par contre, avec les lipides A et B on obtient une amélioration très significative de la cicatrisation par rapport au témoin non traité. Cette activité cicatrisante est supérieure à celle d'une solution d'acide acéxamique à 5 % en poids ou d'une solution de collagène humain à Ø,1 % en poids.

EXEMPLE 2

Des essais comparatifs ont été effectués avec des compositions contenant 8 % en poids de lipides vésiculaires totaux dans l'eau; les vésicules contenant en poids 47,5 % de lipide non-ionique A ou d'alcool chimylique, 47,5 % de cholestérol et 5 % de dicétylphosphate de sodium. L'alcool chimylique est un éther de glycérol de formule :

$$R\text{-}O\text{-}CH_2CHOHCH_2O\text{-}H \text{ avec } R = C_{16}H_{33}$$

et il constitue un agent cicatrisant et anti-inflammatoire connu susceptible de former des vésicules.

Les résultats sont donnés dans le Tableau II ci-après (page 7).

- Tableau I -

| Lipide testé | Diamètre moyen des vésicules | Coefficient (1) de polydispersité en diamètre | Force de rupture en cN | Activité |
|---|---|---|---|---|
| A | 136 nm | 0,28 | 461,5 ± 18,3 | + 52 % |
| B | 132 nm | 0,22 | 495,0 ± 24,0 | + 63 % |
| C | 150 nm | 0,23 | 360,2 ± 25,7 | + 19 % |
| Témoin (sans application) | - | - | 303,7 ± 17,9 | 0 % |

(1) Le coefficient de polydispersité est une mesure caractérisant l'homogénéité des vésicules quant à leur diamètre moyen. Il est égal à 0 si toutes les vésicules ont le même diamètre. Au-delà de 0,4 la polydispersité est telle que la valeur du diamètre moyen des vesicules, calculée par la méthode des cumulants, n'a plus de signification.

- Tableau II -

| Lipide | Diamètre moyen des vésicules | Coefficient de polydispersité en diamètre | Activité |
|---|---|---|---|
| A | 134 nm | 0,23 | + 30 % |
| Alcool chimylique | 180 nm | 0,18 | + 14 % |
| Témoin (aucune application) | - | - | 0 % |

Le résultat avec l'alcool chimylique donne des valeurs non significatives. Par contre, avec le lipide A, on constate une amélioration significative de la cicatrisation.

EXEMPLE 3 : Effet de taille des vésicules et influence du procédé de préparation de celles-ci sur l'activité cicatrisante.

On a effectué trois essais avec des compositions contenant 8 % en poids de lipides vésiculaires totaux dans l'eau; les vésicules étaient préparées à partir de mélanges lipidiques contenant en poids 47,5 % de lipide non-ionique A, 47,5 % de cholestérol et 5 % de dicétylphosphate de sodium.

Les essais 1 et 3 ont été effectués avec des vésicules non-ioniques préparées par un procédé connu, par exemple celui de FR-A-2 315 991, mettant en oeuvre une fusion des lipides par chauffage suivie d'une dispersion dans l'eau par secouage. La dispersion (1) est testée telle quelle, la dispersion (3) est affinée aux ultrasons. L'essai 2 a été effectué avec des vésicules non-ioniques préparées par le procédé avec formation d'un film par évaporation de solvant.

Les résultats sont consignés dans le Tableau III :

- Tableau III -

| Essai | Diamètre moyen des vésicules | Coefficient de polydispersité en diamètre | Activité |
|---|---|---|---|
| 1 | > 1000 nm | hétérogène en taille | + 26 % |
| 2 | 189 nm | 0,33 | + 43 % |
| 3 | 183 nm | 0,31 | + 34 % |
| Témoin (aucune application) | - | - | 0 % |

On note que l'activité des vésicules non-ioniques augmente lorsqu'on diminue la taille des vésicules. Par contre, le procédé de fabrication n'a pas d'influence significative.

EXEMPLE 4

On a mis en solution, à raison de 8 % en poids dans le dipropylèneglycol, un mélange contenant 47,5 % en poids de lipide non-ionique A, 47,5 % en poids de cholestérol et 5 % en poids de dicétylphosphate de sodium. Dans ces conditions, les lipides ne forment pas de vésicules.

L'essai de cicatrisation montre que les lipides non-ioniques de l'invention, seuls ou associés au cholestérol et au dicétyl phosphate, mis en solution dans un solvant, n'ont pas d'activité cicatrisante.

EXEMPLE 5

On a mis en solution, à raison de 3,8 % en poids, dans différents solvants, les lipides non-ioniques A ou B. Dans ces conditions, ils ne forment pas de vésicules. Les essais de cicatrisation ont donné les résultats consignés dans le tableau IV.

On obtient une activité de 0 % comme c'est d'ailleurs aussi le cas avec des animaux traités avec le solvant seul.

- Tableau IV -

| Lipide | Solvant | Activité |
|---|---|---|
| A | Méthyléther du dipropylène glycol (*) | Ø % |
| B | Méthyléther du dipropylène glycol (*) | Ø % |
| A | Dipropylène glycol | Ø % |
| Rien | Méthyléther du dipropylène glycol | Ø % |
| Rien | Dipropylène glycol | Ø % |
| Témoin (aucune application) | - | Ø % |

(*) Vendu par la Société "DOW CHEMICAL" sous la dénomination commerciale "DOWANOL DPM".

Ces essais montrent que, lorsque les lipides non-ioniques A et B ne sont pas sous forme de vésicules, ils n'ont pas d'action cicatrisante.

EXEMPLE 6 : Préparation d'une lotion cicatrisante.

Dans un bécher en acier inoxydable, on pèse les produits :

- Lipide amphiphile non-ionique de formule A défini dans l'exemple 1 3,8 g
- Cholestérol 3,8 g
- Dicétyl phosphate de sodium 0,4 g

On réalise le mélange de ces trois produits par fusion à la température de 11Ø°C sous atmosphère d'azote, puis on ramène la température du mélange fondu à 9Ø°C.

On ajoute 20 g d'eau déminéralisée. On homogénéise le mélange obtenu à la température de 9Ø°C. On ajoute alors Ø,3 g de parahydroxybenzoate de méthyle dissous dans 51,3 g d'eau déminéralisée.

A la température de 7Ø°C, on homogénéise le mélange à l'aide d'un ultradisperseur "Virtis" jusqu'à ce que la taille moyenne des vésicules obtenues soit de 200 nm.

On ajoute enfin les substances suivantes :

- Mélange d'acides carboxyvinyliques commercialisé sous la dénomination commerciale "Carbopol 94Ø" par la Société GOODRICH Ø,2 g
- Triéthanolamine Ø,2 g
- Eau déminéralisée 2Ø,Ø g

La lotion ci-dessus est appliquée sur une plaie à raison de 3 mg/ jour/cm2. On observe qu'au bout de 12 jours, la phase aiguë de la cicatrisation est terminée.

EXEMPLE 7 : Gel cicatrisant

Dans un bécher en acier inoxydable, on pèse les produits suivants :
- Lipide amphiphile non-ionique de formule A défini dans l'exemple 1. 5,Ø g
- Cholestérol 5,Ø g

On réalise le mélange de ces deux produits par fusion à la température de 11Ø°C sous atmosphère d'azote, puis on ramène la température du mélange fondu à 9Ø°C. On ajoute alors 2Ø g d'eau déminéralisée.

On homogénéise le mélange obtenu à la température de 9Ø°C. On ajoute alors Ø,3 g de parahydroxybenzoate de méthyle dissous dans 48,88 g d'eau déminéralisée.

A la température de 7Ø°C, on homogénéise le mélange à l'aide d'un ultradisperseur "Virtis" jusqu'à ce que la taille moyenne des vésicules obtenues soit de 500 nm.

On ajoute enfin les substances suivantes :
- Mélange d'acides carboxyvinyliques commercialisé sous le nom commercial de "Carbopol 94Ø" par la Société GOODRICH Ø,42 g
- Triéthanolamine Ø,4 g
- Eau déminéralisée 2Ø g

Le gel ci-dessus est appliqué sur une plaie à raison de 2 mg/jour/ cm2. On observe qu'au bout de 12 jours, la phase aiguë de la cicatrisation est terminée.

EXEMPLE 8 : Lotion cicatrisante

Dans un bécher en acier inoxydable, on pèse les produits :
- Lipide amphiphile non-ionique de formule A défini dans l'exemple 1. 3,25 g
- Cholestérol 1,25 g
- Dicétyl phosphate de sodium Ø,5Ø g

On réalise le mélange de ces trois produits par fusion à la température de 11Ø°C sous atmosphère d'azote, puis on ramène la température du mélange à 9Ø°C. On ajoute alors 1Ø,Ø g d'eau déminéralisée.

On homogénéise le mélange obtenu à la température de 9Ø°C. On ajoute alors Ø,3 g de parahydroxybenzoate de méthyle dissous dans 84,7 g d'eau déminéralisée.

A la température de 7Ø°C, on homogénéise le mélange à l'aide d'un ultradisperseur "Virtis" jusqu'à ce que la taille moyenne des vésicules obtenues soit de 100 nm.

La dispersion obtenue est fluide et peut être appliquée à partir d'un flacon pompe.

La lotion ci-dessus est appliquée sur une plaie à raison de 4 mg/ jour/cm2. On observe qu'au bout de 12 jours, la phase aiguë de la cicatrisation est terminée.

EXEMPLE 9 : Lotion cicatrisante

Dans un ballon rond en verre de 1 litre, on dissout, dans 200 ml d'un mélange de solvants chloroforme/méthanol dans le rapport 2/1, les produits suivants :
- Lipide amphiphile non-ionique de formule B défini dans l'exemple 1. 3,8 g
- Cholestérol 3,8 g
- Dicétyl phosphate de sodium Ø,4 g

On évapore ensuite les solvants à l'aide d'un évaporateur rotatif et on élimine les dernières traces de solvants par mise sous la pression réduite d'une pompe à palettes pendant une heure. On met en contact l'association de lipides obtenue avec 2Ø g d'eau déminéralisée mélangée à 3 g de glycérine.

On homogénéise le mélange à la température de 9Ø°C. On ajoute ensuite :
- Parahydroxybenzoate de méthyle 0,3 g
- Eau déminéralisée 48,3 g

On soumet le tout à l'action d'un ultra-disperseur du type "Virtis" jusqu'à ce que la taille des vésicules obtenues soit inférieure à Ø,2 micron.

On ajoute enfin les substances suivantes :

- Mélange d'acides carboxyvinyliques commercialisé sous la dénomination commerciale "Carbopol 94Ø" par la Société GOODRICH Ø,2 g
- Triéthanolamine Ø,2 g
- Eau déminéralisée 2Ø,Ø g

La lotion ci-dessus est appliquée sur une plaie à raison de 3 mg/jour /cm2. On observe qu'au bout de 12 jours, la phase aiguë de la cicatrisation est terminée.

EXEMPLE 1Ø : Gel cicatrisant

Dans un bécher en acier inoxydable, on pèse les produits suivants :

- Lipide amphiphile non-ionique de formule A 11,875 g
- Cholestérol 11,875 g
- Dicétyl phosphate de sodium 1,25Ø g

On réalise le mélange de ces trois produits par fusion à la température de 11Ø°C sous atmosphère d'azote, puis on ramène la température du mélange fondu à 9Ø°C. On ajoute 5Ø g d'eau déminéralisée.

On homogénéise le mélange obtenu à la température de 9Ø°C. On ajoute alors Ø,3 g de parahydroxybenzoate de méthyle dissous dans 24,7 g d'eau déminéralisée.

A la température de 7Ø°C, on homogénéise le mélange à l'aide d'un ultradisperseur "Virtis" jusqu'à ce que la taille moyenne des vésicules obtenues soit inférieure à 1000 nm.

Le gel ci-dessus est appliqué sur une plaie à raison de 2 mg/ jour/cm2. On observe qu'au bout de 12 jours, la phase aiguë de la cicatrisation est terminée.

## Revendications

### Revendications pour les Etats contractants suivants : AT, BE, CH, DE, DK, GB, IT, LI, NL, SE

**1.** Composition pour application topique sur la peau comportant dans une phase aqueuse D, des vésicules encapsulant une phase aqueuse E, lesdites vésicules étant obtenues à partir d'au moins un lipide non-ionique dérivé de polyglycérol associé à du cholestérol, la concentration des lipides vésiculaires dans la composition étant comprise entre 5 et 25 % en poids, caractérisée par le fait que, pour conférer à ladite composition une action cicatrisante en l'absence de tout agent de cicatrisation dans les phases aqueuses E ou D, le(s) dérivé(s) de polyglycérol est(sont) choisi(s) dans le groupe formé par ceux de formule:

$$RO(CH_2\text{-}CHOH\text{-}CH_2O)_2H \qquad (I)$$

formule dans laquelle R représente soit un radical alkyle en $C_{16}$-$C_{18}$ ou un mélange de plusieurs de ces radicaux, soit un radical R'CO-, R' étant un radical alkyle en $C_{15}$-$C_{17}$ ou un mélange de plusieurs de ces radicaux, ledit lipide non-ionique étant associé à du cholestérol et la concentration des lipides vésiculaires totaux dans la composition étant de 5 a 25 % en poids.

**2.** Composition selon la revendication 1, caractérisée par le fait qu'un dicétylphosphate ou un tétracétylphosphate est également associé au(x) lipide(s) non-ionique(s) de formule(I).

**3.** Composition selon l'une des revendications 1 ou 2, caractérisée par le fait que les vésicules sont obtenues à partir d'un mélange de lipides contenant en poids 40 à 75 % de lipides de formule (I), 20 à 50 % de cholestérol et 0 à 10 % d'un dicétylphosphate.

**4.** Composition selon l'une des revendications 1 à 3, caractérisée par le fait que les vésicules ont des diamètres moyens compris entre 25 et 5000 nm.

**5.** Composition selon l'une des revendications 1 à 4, caractérisée par le fait que les vésicules ont un coefficient de polydispersité inférieur à 0,4 %.

**6.** Composition selon l'une des revendications 1 à 5, caractérisée par le fait que la phase aqueuse E encapsulée dans les vésicules et la phase de dispersion D sont identiques.

**7.** Composition selon l'une des revendications 1 à 6, caractérisée par le fait que la phase aqueuse E et/ou la phase aqueuse D contiennent au moins un additif cosmétique et/ou au moins un additif pharmaceutiquement actif.

8. Composition selon l'une des revendications 1 à 7, caractérisée par le fait que les feuillets lipidiques des vésicules contiennent au moins un additif liposoluble cosmétique et/ou au moins un additif liposoluble pharmaceutiquement actif.

9. Utilisation, pour favoriser la cicatrisation, d'une composition contenant, dispersées dans une phase aqueuse D, des vésicules obtenues à partir d'au moins un lipide non-ionique de formule

$$RO\{CH_2\text{-}CHOH\text{-}CH_2O\}_{\bar{2}}H \quad (I)$$

formule dans laquelle R représente soit un radical alkyle en $C_{16}$ - $C_{18}$ ou un mélange de plusieurs de ces radicaux, soit un radical R' CO-, R' étant au moins un radical alkyle en $C_{15}$ - $C_{17}$ ou un mélange de plusieurs de ces radicaux, ledit lipide non-ionique étant associé à du cholestérol et la concentration des lipides vésiculaires totaux dans la composition étant de 5 à 25 % en poids.

10. Utilisation selon la revendication 9, caractérisée par le fait qu'un dicétylphosphate ou un tétracétylphosphate est également associé au(x) lipide(s) non-ionique(s) de formule (I).

11. Utilisation selon l'une des revendications 9 ou 10, caractérisée par le fait que les vésicules sont obtenues à partir d'un mélange de lipides contenant en poids 40 à 75 % de lipides de formule (I), 20 à 50 % de cholestérol et 0 à 10 % d'un dicétylphosphate.

12. Utilisation selon l'une des revendications 9 à 11, caractérisée par le fait que les vésicules ont des diamètres moyens compris entre 25 et 5000 nm.

13. Utilisation selon l'une des revendications 9 à 12, caractérisée par le fait que les vésicules ont un coefficient de polydispersité inférieur à 0,4.

14. Utilisation selon l'une des revendications 9 à 13, caractérisée par le fait que la phase aqueuse E encapsulée dans les vésicules et la phase de dispersion D sont identiques.

15. Utilisation selon l'une des revendications 9 à 14, caractérisée par le fait que la phase aqueuse E et/ou la phase aqueuse D contiennent au moins un additif cosmétique et/ou au moins un additif pharmaceutiquement actif.

16. Utilisation selon l'une des revendications 9 à 15, caractérisée par le fait que les feuillets lipidiques des vésicules contiennent au moins un additif liposoluble cosmétique et/ou au moins un additif liposoluble pharmaceutiquement actif.

17. Utilisation selon l'une des revendications 9 à 16, caractérisée par le fait que la composition est appliquée sur la peau à raison de 2 à 8 mg/jour/$cm^2$.

**Revendications pour les Etats contractants suivants : ES, GR**

1. Procédé de préparation d'une composition pour application topique sur la peau comportant dans une phase aqueuse D, des vésicules encapsulant une phase aqueuse E, lesdites vésicules étant obtenues à partir d'au moins un lipide non-ionique dérivé de polyglycérol caractérisé par le fait qu'on mélange du cholestérol et au moins un lipide non-ionique de formule:

$$RO\{CH_2\text{-}CHOH\text{-}CH_2O\}_{\bar{2}}H \quad (I)$$

formule dans laquelle R représente soit un radical alkyle en $C_{16}$-$C_{18}$ ou un mélange de plusieurs de ces radicaux, soit un radical R'CO, R' étant au moins un radical alkyle en $C_{15}$-$C_{17}$ ou un mélange de ces radicaux, de façon à obtenir une phase lipidique, que l'on prépare à partir de cette phase lipidique par contact avec une phase aqueuse, des vésicules encapsulant une phase aqueuse E sous forme de dispersion dans une phase aqueuse D et que l'on ajuste la concentration des lipides vésiculaires totaux dans la dispersion à une valeur comprise entre 5 et 25 % en poids.

2. Procédé selon la revendication 1, caractérisé par le fait qu'on ajuste la concentration des lipides vésiculaires totaux à une valeur comprise entre 6 et 10 %.

**3.** Procédé selon la revendication 1, caractérisé par le fait que pour obtenir la phase lipidique on mélange, avec le cholestérol et au(x) lipide(s) non-ionique(s) de formule I, un dicétyl phosphate ou un tétracétyl phosphate.

**4.** Procédé selon l'une des revendications 1 à 3, caractérisé par le fait que pour obtenir la phase lipidique on mélange en poids, 40 à 75 % de lipide(s) de formule I, 20 à 50 % de cholestérol et 0 à 10 % de dicétyl phosphate.

**5.** Procédé selon l'une des revendications 1 à 4, caractérisé par le fait qu'on ajoute dans la phase aqueuse avant formation des vésicules au moins un additif cosmétique et/ou au moins un additif pharmaceutiquement actif.

**6.** Procédé selon l'une des revendications 1 à 4, caractérisé par le fait qu'on ajoute à la phase aqueuse de dispersion D, après formation des vésicules, au moins un addtif cosmétique et/ou au moins un additif pharmaceutiquement actif.

**7.** Procédé selon l'une des revendications 1 à 6, caractérisé par le fait qu'on ajoute à la phase lipidique au moins un additif liposoluble cosmétique et/ou au moins un additif liposoluble pharmaceutiquement actif.

**8.** Procédé selon l'une des revendications 1 à 7, caractérisée par le fait que la phase aqueuse E encapsulée dans les vésicules et la phase de dispersion D sont identiques.

**9.** Procédé selon l'une des revendications 1 à 8, caractérisée par le fait que la phase aqueuse E et/ou la phase aqueuse D contiennent au moins un additif cosmétique et/ou au moins un additif pharmaceutiquement actif.

**10.** Procédé selon l'une des revendications 1 à 9, caractérisée par le fait que les feuillets lipidiques des vésicules contiennent au moins un additif liposoluble cosmétique et/ou au moins un additif liposoluble pharceutiquement actif.

**11.** Utilisation, pour l'obtention d'un médicament favorisant la cicatrisation, d'une composition contenant, dispersées dans une phase aqueuse D, des vésicules obtenues à partir d'au moins un lipide non-ionique de formule:

$$RO\{CH_2-CHOH-CH_2O\}_{2}H \quad (I)$$

formule dans laquelle R représente soit un radical alkyle en $C_{16}$ - $C_{18}$ ou un mélange de plusieurs de ces radicaux, soit un radical R'CO-, R' étant au moins un radical alkyle en $C_{15}$ - $C_{17}$ ou un mélange de plusieurs de ces radicaux, ledit lipide non-ionique étant associé à du cholestérol et la concentration des lipides vésiculaires totaux dans la composition étant de 5 à 25 % en poids.

**12.** Utilisation selon la revendication 11, caractérisée par le fait qu'un dicétylphosphate ou un tétracétylphosphate est également associé au(x) lipide(s) non-ionique(s) de formule (I).

**13.** Utilisation selon l'une des revendications 11 ou 12, caractérisée par le fait que les vésicules sont obtenues à partir d'un mélange de lipides contenant en poids 40 à 75 % de lipides de formule (I), 20 à 50 % de cholestérol et 0 à 10 % d'un dicétylphosphate.

**14.** Utilisation selon l'une des revendications 11 à 13, caractérisée par le fait que les vésicules ont des diamètres moyens compris entre 25 et 5 000 nm.

**15.** Utilisation selon l'une des revendications 11 à 14, caractérisée par le fait que les vésicules ont un coefficient de polydispersité inférieur à 0,4.

**16.** Utilisation selon l'une des revendications 11 à 15, caractérisée par le fait que la phase aqueuse E encapsulée dans les vésicules et la phase de dispersion D sont identiques.

**17.** Utilisation selon l'une des revendications 11 à 16, caractérisée par le fait que la phase aqueuse E et/ou la phase aqueuse D contiennent au moins un additif cosmétique et/ou au moins un additif pharmaceutiquement actif.

**18.** Utilisation selon l'une des revendications 11 à 17, caractérisée par le fait que les feuillets lipidiques des vésicules contiennent au moins un additif liposoluble cosmétique et/ou au moins un additif liposoluble pharmaceutiquement actif.

## Claims

### Claims for the following Contracting States : AT, BE, CH, DE, DK, GB, IT, LI, NL, SE

**1.** Composition for topical application to the skin, containing, in an aqueous phase D, vesicles encapsulating an aqueous phase E, the said vesicles being obtained from at least one nonionic lipid derived from polyglycerol combined with cholesterol, the concentration of the vesicular lipids in the composition being between 5 and 25 % by weight, characterized in that, to endow the said composition with a cicatrizing action in the absence of any cicatrizing agent in the aqueous phases E or D, the polyglycerol derivative(s) is/are chosen from the group composed of those of formula:

$$RO\{CH_2\text{-}CHOH\text{-}CH_2O\}_{2}H \qquad (I)$$

in which formula R either represents a $C_{16}$-$C_{18}$ alkyl radical or a mixture of several of these radicals, or represents a radical R' CO-, R' being a $C_{15}$-$C_{17}$ alkyl radical or a mixture of several of these radicals, the said nonionic lipid being combined with cholesterol and the concentration of total vesicular lipids in the composition being from 5 to 25 % by weight.

**2.** Composition according to Claim 1, characterized in that a dicetyl phosphate or a tetracetyl phosphate is also combined with the nonionic lipid(s) of formula (I).

**3.** Composition according to one of Claims 1 and 2, characterized in that the vesicles are obtained from a mixture of lipids containing, by weight, 40 to 75 % of lipids of formula (I), 20 to 50 % of cholesterol and 0 to 10 % of a dicetyl phosphate.

**4.** Composition according to one of Claims 1 to 3, characterized in that the vesicles have average diameters of between 25 and 5000 nm.

**5.** Composition according to one of Claims 1 to 4, characterized in that the vesicles have a coefficient of polydispersity of less than 0.4 %.

**6.** Composition according to one of Claims 1 to 5, characterized in that the aqueous phase E encapsulated in the vesicles and the dispersion phase D are identical.

**7.** Composition according to one of Claims 1 to 6, characterized in that the aqueous phase E and/or the aqueous phase D contain at least one cosmetic additive and/or at least one pharmaceutically active additive.

**8.** Composition according to one of Claims 1 to 7, characterized in that the lipid lamellae of the vesicles contain at least one fat-soluble cosmetic additive and/or at least one fat-soluble pharmaceutically active additive.

**9.** Use for promoting cicatrization of a composition containing, dispersed in an aqueous phase D, vesicles obtained from at least one nonionic lipid of formula:

$$RO\{CH_2\text{-}CHOH\text{-}CH_2O\}_{2}H \qquad (I)$$

in which formula R either represents a $C_{16}$-$C_{18}$ alkyl radical or a mixture of several of these radicals, or represents a radical R'CO-, R' being at least one $C_{15}$-$C_{17}$ alkyl radical or a mixture of several of these radicals, the said nonionic lipid being combined with cholesterol and the concentration of total vesicular lipids in the composition being from 5 to 25 % by weight.

**10.** Use according to Claim 9, characterized in that a dicetyl phosphate or a tetracetyl phosphate is also combined with the nonionic lipid(s) of formula (I).

**11.** Use according to one of Claims 9 and 10, characterized in that the vesicles are obtained from a mixture

of lipids containing, by weight, 40 to 75 % of lipids of formula (I), 20 to 50 % of cholesterol and 0 to 10 % of a dicetyl phosphate.

**12.** Use according to one of Claims 9 to 11, characterized in that the vesicles have average diameters of between 25 and 5000 nm.

**13.** Use according to one of Claims 9 to 12, characterized in that the vesicles have a coefficient of polydispersity of less than 0.4.

**14.** Use according to one of Claims 9 to 13, characterized in that the aqueous phase E encapsulated in the vesicles and the dispersion phase D are identical.

**15.** Use according to one of Claims 9 to 14, characterized in that the aqueous phase E and/or the aqueous phase D contain at least one cosmetic additive and/or at least one pharmaceutically active additive.

**16.** Use according to one of Claims 9 to 15, characterized in that the lipid lamellae of the vesicles contain at least one fat-soluble cosmetic additive and/or at least one fat-soluble pharmaceutically active additive.

**17.** Use according to one of Claims 9 to 16, characterized in that the composition is applied to the skin on the basis of 2 to 8 $mg/day/cm^2$.

**Claims for the following Contracting States : ES, GR**

**1.** Process for preparing a composition for topical application to the skin, containing, in an aqueous phase D, vesicles encapsulating an aqueous phase E, the said vesicles being obtained from at least one nonionic lipid derived from polyglycerol, characterized in that cholesterol and at least one nonionic lipid of formula:

$$RO\text{-}(CH_2\text{-}CHOH\text{-}CH_2O)_{2}\,H \qquad (I)$$

in which formula R either represents a $C_{16}$-$C_{18}$ alkyl radical or a mixture of several of these radicals, or represents a radical R'CO, R' being at least one $C_{15}$-$C_{17}$ alkyl radical or a mixture of these radicals, are mixed so as to obtain a lipid phase, in that, by contact with an aqueous phase, vesicles encapsulating an aqueous phase E are prepared from this lipid phase in the form of a dispersion in an aqueous phase D, and in that the concentration of total vesicular lipids in the dispersion is adjusted to a value of between 5 and 25 % by weight.

**2.** Process according to Claim 1, characterized in that the concentration of total vesicular lipids is adjusted to a value of between 6 and 10 %.

**3.** Process according to Claim 1, characterized in that, to obtain the lipid phase, a dicetyl phosphate or a tetracetyl phosphate is mixed with cholesterol and the nonionic lipid(s) of formula (I).

**4.** Process according to one of Claims 1 to 3, characterized in that, to obtain the lipid phase, 40 to 75 % of lipid(s) of formula (I), 20 to 50 % of cholesterol and 0 to 10 % of dicetyl phosphate, these percentages being by weight, are mixed.

**5.** Process according to one of Claims 1 to 4, characterized in that at least one cosmetic additive and/or at least one pharmaceutically active additive is/are added to the aqueous phase before formation of the vesicles.

**6.** Process according to one of Claims 1 to 4, characterized in that at least one cosmetic additive and/or at least one pharmaceutically active additive is/are added to the aqueous dispersion phase D after formation of the vesicles.

**7.** Process according to one of Claims 1 to 6, characterized in that at least one fat-soluble cosmetic additive and/or at least one fat-soluble pharmaceutically active additive is/are added to the lipid phase.

**8.** Process according to one of Claims 1 to 7, characterized in that the aqueous phase E encapsulated in the vesicles and the dispersion phase D are identical.

9. Process according to one of Claims 1 to 8, characterized in that the aqueous phase E and/or the aqueous phase D contain at least one cosmetic additive and/or at least one pharmaceutically active additive.

10. Process according to one of Claims 1 to 9, characterized in that the lipid lamellae of the vesicles contain at least one fat-soluble cosmetic additive and/or at least one fat-soluble pharmaceutically active additive.

11. Use, for obtaining a medicinal product which promotes cicatrization, of a composition containing, dispersed in an aqueous phase D, vesicles obtained from at least one nonionic lipid of formula:

$$RO\{CH_2\text{-}CHOH\text{-}CH_2O\}_{2}H \qquad (I)$$

in which formula R either represents a $C_{16}$-$C_{18}$ alkyl radical or a mixture of several of these radicals, or represents a radical R'CO-, R' being at least one $C_{15}$-$C_{17}$ alkyl radical or a mixture of several of these radicals, the said nonionic lipid being combined with cholesterol and the concentration of total vesicular lipids in the composition being from 5 to 25 % by weight.

12. Use according to Claim 11, characterized in that a dicetyl phosphate or a tetracetyl phosphate is also combined with the nonionic lipid(s) of formula (I).

13. Use according to one of Claims 11 and 12, characterized in that the vesicles are obtained from a mixture of lipids containing, by weight, 40 to 75 % of lipids of formula (I), 20 to 50 % of cholesterol and 0 to 10 % of a dicetyl phosphate.

14. Use according to one of Claims 11 to 13, characterized in that the vesicles have average diameters of between 25 and 5000 nm.

15. Use according to one of Claims 11 to 14, characterized in that the vesicles have a coefficient of polydispersity of less than 0.4.

16. Use according to one of Claims 11 to 15, characterized in that the aqueous phase E encapsulated in the vesicles and the dispersion phase D are identical.

17. Use according to one of Claims 11 to 16, characterized in that the aqueous phase E and/or the aqueous phase D contain at least one cosmetic additive and/or at least one pharmaceutically active additive.

18. Use according to one of Claims 11 to 17, characterized in that the lipid lamellae of the vesicles contain at least one fat-soluble cosmetic additive and/or at least one fat-soluble pharmaceutically active additive.

**Patentansprüche**

**Patentansprüche für folgende Vertragsstaaten : AT, BE, CH, DE, DK, GB, IT, LI, NL, SE**

1. Zusammensetzung zur topischen Anwendung auf der Haut, welche in einer wäßrigen Phase D Vesikel umfaßt, die eine wäßrige Phase E einkapseln, wobei die Vesikel ausgehend von einem nicht-ionischen, von Polyglycerin abgeleiteten und mit Cholesterin assoziierten Lipid hergestellt wurden und wobei die Konzentration der Vesikellipide in der Zusammensetzung zwischen 5 und 25 Gew.-% liegt, dadurch gekennzeichnet, daß, um der Zusammensetzung eine narbenbildende Wirkung in Abwesenheit eines jeden narbenbildenden Mittels in den wäßrigen Phasen E oder D zu verleihen, das Polyglycerinderivat (die Polyglycerinderivate) ausgewählt ist (sind) unter denjenigen der Formel:

$$RO\{CH_2\text{-}CHOH\text{-}CH_2O\}_{2}H$$

worin R für einen $C_{16}$-$C_{18}$-Alkylrest oder eine Mischung von mehreren dieser Reste oder einen Rest R'CO- steht, wobei R' einen $C_{15}$-$C_{17}$-Alkylrest oder eine Mischung aus mehreren dieser Reste bedeutet, wobei das nicht-ionische Lipid mit Cholesterin assoziiert ist und die Konzentration der gesamten Vesikellipide in der Zusammensetzung 5 bis 25 Gew.-% beträgt.

2. Zusammensetzung nach Anspruch 1, dadurch gekennzeichnet, daß auch ein Dicetylphosphat oder ein Tetracetylphosphat mit dem nicht-ionischen Lipid (oder den nicht-ionischen Lipiden) der Formel (I) assoziiert ist.

3. Zusammensetzung nach einem der Ansprüche 1 oder 2, dadurch gekennzeichnet, daß die Vesikel ausgehend von einem Lipidgemisch erhalten wurden, das 40 bis 75 Gew.-% Lipide der Formel (I), 20 bis 50 Gew.-% Cholesterin und 0 bis 10 Gew.-% eines Dicetylphosphates enthält.

4. Zusammensetzung nach einem der Ansprüche 1 bis 3, dadurch gekennzeichnet, daß die Vesikel einen mittleren Durchmesser zwischen 25 und 5000 nm aufweisen.

5. Zusammensetzung nach einem der Ansprüche 1 bis 4, dadurch gekennzeichnet, daß die Vesikel einen PolydispersitätsKoeffizienten von weniger als 0,4 % aufweisen.

6. Zusammensetzung nach einem der Ansprüche 1 bis 5, dadurch gekennzeichnet, daß die in den Vesikeln eingekapselte wäßrige Phase E und die Dispersionsphase D identisch sind.

7. Zusammensetzung nach einem der Ansprüche 1 bis 6, dadurch gekennzeichnet, daß die wäßrige Phase E und/oder die wäßrige Phase D wenigstens ein kosmetisches Additiv und/oder wenigstens ein pharmazeutisch wirksames Additiv enthalten.

8. Zusammensetzung nach einem der Ansprüche 1 bis 7, dadurch gekennzeichnet, daß die Lipidblättchen der Vesikel wenigstens ein fettlösliches kosmetisches Additiv und/oder wenigstens ein fettlösliches, pharmazeutisch wirksames Additiv enthalten.

9. Verwendung einer Zusammensetzung, die Vesikel in einer wäßrigen Phase D dispergiert enthält, welche ausgehend von einem nicht-ionischen Lipid der Formel

$$RO\{CH_2-CHOH-CH_2O\}_2H \qquad (I)$$

worin R für einen $C_{16}$-$C_{18}$-Alkylrest oder eine Mischung aus mehreren dieser Reste oder einen Rest R'CO- steht, wobei R'-wenigstens einen $C_{15}$-$C_{17}$-Alkylrest oder eine Mischung aus mehreren dieser Reste bedeutet, wobei das nicht-ionische Lipid mit Cholesterin assoziiert ist und die Konzentration der gesamten Vesikellipide in der Zusammensetzung 5 bis 25 Gew.-% beträgt, zur Förderung der Narbenbildung.

10. Verwendung nach Anspruch 9, dadurch gekennzeichnet, daß auch ein Dicetylphosphat oder ein Tetracetylphosphat mit dem nicht-ionischen Lipid (oder den nicht-ionischen Lipiden) der Formel I assoziiert ist.

11. Verwendung nach einem der Ansprüche 9 oder 10, dadurch gekennzeichnet, daß die Vesikel ausgehend von einem Lipidgemisch erhalten wurden, das 40 bis 75 Gew.-% Lipide der Formel (I), 20 bis 50 Gew.-% Cholesterin und 0 bis 10 Gew.-% eines Dicetylphosphates enthält.

12. Verwendung nach einem der Ansprüche 9 bis 11, dadurch gekennzeichnet, daß die Vesikel einen mittleren Durchmesser zwischen 25 und 5000 nm aufweisen.

13. Verwendung nach einem der Ansprüche 9 bis 12, dadurch gekennzeichnet, daß die Vesikel einen Polydispersitäts-Koeffizienten von weniger als 0,4 aufweisen.

14. Verwendung nach einem der Ansprüche 9 bis 13, dadurch gekennzeichnet, daß die in die Vesikel eingekapselte wäßrige Phase E und die Dispersionsphase D identisch sind.

15. Verwendung nach einem der Ansprüche 9 bis 14, dadurch gekennzeichnet, daß die wäßrige Phase E und/oder die wäßrige Phase D wenigstens ein kosmetisches Additiv und/oder wenigstens ein pharmazeutisch wirksames Additiv enthalten.

16. Verwendung nach einem der Ansprüche 9 bis 15, dadurch gekennzeichnet, daß die Lipidblättchen der Vesikel wenigstens ein fettlösliches kosmetisches Additiv und/oder wenigstens ein fettlösliches pharmazeutisch wirksames Additiv enthalten.

17. Verwendung nach einem der Ansprüche 9 bis 16, dadurch gekennzeichnet, daß die Zusammensetzung in einer Menge von 2 bis 8 mg/Tag/$cm^2$ auf die Haut aufgetragen wird.

**Patentansprüche für folgende Vertragsstaaten : ES, GR**

1. Verfahren zur Herstellung einer Zusammensetzung zur topischen Anwendung auf der Haut, die in einer wäßrigen Phase D Vesikel umfaßt, welche eine wäßrige Phase E einkapseln, wobei die Vesikel ausgehend von wenigstens einem nicht-ionischen, von Polyglycerin abgeleiteten Lipid hergestellt wurden, dadurch gekennzeichnet, daß man Cholesterin und wenigstens ein nichtionisches Lipid der Formel:

$$RO{-}(CH_2{-}CHOH{-}CH_2O)_2{-}H$$

worin R für einen $C_{16}$-$C_{18}$-Alkylrest oder eine Mischung aus mehreren dieser Reste, oder einen Rest R'CO- steht, wobei R' wenigstens einen $C_{15}$-$C_{17}$-Alkylrest oder eine Mischung dieser Reste bedeutet, so vermischt, daß man eine Lipidphase erhält, daß man ausgehend von dieser Lipidphase durch Inkontaktbringen mit einer wäßrigen Phase Vesikel, welche eine wäßrige Phase E einkapseln, in Form einer Dispersion in einer wäßrigen Phase D herstellt und daß man die Konzentration der gesamten Vesikellipide in der Dispersion auf einen Wert zwischen 5 und 25 Gew.-% einstellt.

2. Verfahren nach Anspruch 1, dadurch gekennzeichnet, daß man die Konzentration der gesamten Vesikellipide auf einen Wert zwischen 6 und 10 % einstellt.

3. Verfahren nach Anspruch 1, dadurch gekennzeichnet, daß man für die Herstellung der Lipidphase ein Dicetylphosphat oder ein Tetracetylphosphat mit Cholesterin und dem nicht-ionischen Lipid (den nichtionischen Lipiden) der Formel I vermischt.

4. Verfahren nach einem der Ansprüche 1 bis 3, dadurch gekennzeichnet, daß man zur Herstellung der Lipidphase 40 bis 75 Gew.-% Lipid(e) der Formel I, 20 bis 50 Gew.-% Cholesterin und 0 bis 10 Gew.-% Dicetylphosphat vermischt.

5. Verfahren nach einem der Ansprüche 1 bis 4, dadurch gekennzeichnet, daß man vor Bildung der Vesikel wenigstens ein kosmetisches und/oder wenigstens ein pharmazeutisch wirksames Additiv in die wäßrige Phase gibt.

6. Verfahren nach einem der Ansprüche 1 bis 4, dadurch gekennzeichnet, daß man nach Bildung der Vesikel wenigstens ein kosmetisches Additiv und/oder wenigstens ein pharmazeutisch wirksames Additiv zur wäßrigen Phase der Dispersion D gibt.

7. Verfahren nach einem der Ansprüche 1 bis 6, dadurch gekennzeichnet, daß man wenigstens ein fettlösliches kosmetisches Additiv und/oder wenigstens ein fettlösliches pharmazeutisch wirksames Additiv zur Lipidphase gibt.

8. Verfahren nach einem der Ansprüche 1 bis 7, dadurch gekennzeichnet, daß die in die Vesikel eingekapselte wäßrige Phase E und die Dispersionsphase D identisch sind.

9. Verfahren nach einem der Ansprüche 1 bis 8, dadurch gekennzeichnet, daß die wäßrige Phase E und/oder die wäßrige Phase D wenigstens ein kosmetisches Additiv und/oder wenigstens ein pharmazeutisch wirksames Additiv enthalten.

10. Verfahren nach einem der Ansprüche 1 bis 9, dadurch gekennzeichnet, daß die Lipidblättchen der Vesikel wenigstens ein fettlösliches kosmetisches Additiv und/oder wenigstens ein fettlösliches pharmazeutisch wirksames Additiv enthalten.

11. Verwendung einer Zusammensetzung, die Vesikel in einer wäßrigen Phase D dispergiert enthält, welche ausgehend von einem nicht-ionischen Lipid der Formel

$$RO{-}(CH_2{-}CHOH{-}CH_2O)_2{-}H$$

worin R für einen $C_{16}$-$C_{18}$-Alkylrest oder eine Mischung aus mehreren dieser Reste oder einen Rest R'CO- steht, wobei R' wenigstens einen $C_{15}$-$C_{17}$-Alkylrest oder eine Mischung aus mehreren dieser Reste bedeutet, wobei das nicht-ionische Lipid mit Cholesterin assoziiert ist und die Konzentration der gesamten Vesikellipide in der Zusammensetzung 5 bis 25 Gew.-% beträgt, zur Herstellung eines die Narbenbildung

fördernden Medikamentes.

12. Verwendung nach Anspruch 11, dadurch gekennzeichnet, daß auch ein Dicetylphosphat oder ein Tetracetylphosphat mit dem nicht-ionischen Lipid (oder den nicht-ionischen Lipiden) der Formel I assoziiert ist.

13. Verwendung nach einem der Ansprüche 11 oder 12, dadurch gekennzeichnet, daß die Vesikel ausgehend von einem Lipidgemisch erhalten wurden, das 40 bis 75 Gew.-% Lipide der Formel (I), 20 bis 50 Gew.-% Cholesterin und 0 bis 10 Gew.-% eines Dicetylphosphates enthält.

14. Verwendung nach einem der Ansprüche 11 bis 13, dadurch gekennzeichnet, daß die Vesikel einen mittleren Durchmesser zwischen 25 und 5000 nm aufweisen.

15. Verwendung nach einem der Ansprüche 11 bis 14, dadurch gekennzeichnet, daß die Vesikel einen Polydispersitäts-Koeffizienten von weniger 0,4 aufweisen.

16. Verwendung nach einem der Ansprüche 11 bis 15, dadurch gekennzeichnet, daß die in die Vesikel eingekapselte wäßrige Phase E und die Dispersionsphase D identisch sind.

17. Verwendung nach einem der Ansprüche 11 bis 16, dadurch gekennzeichnet, daß die wäßrige Phase E und/oder die wäßrige Phase D wenigstens ein kosmetisches Additiv und/oder wenigstens ein pharmazeutisch wirksames Additiv enthalten.

18. Verwendung nach einem der Ansprüche 11 bis 17, dadurch gekennzeichnet, daß die Lipidblättchen der Vesikel wenigstens ein fettlösliches kosmetisches Additiv und/oder wenigstens ein fettlösliches pharmazeutisch wirksames Additiv enthalten.